# EUROPEAN PATENT SPECIFICATION

(11) **EP 3 618 782 B1**
(45) Date of publication and mention of the grant of the patent: **26.05.2021**
(21) Application number: 18725414.9
(22) Date of filing: 03.05.2018
(51) Int. Cl.: A61F 9/00

(54) **DROP DISPENSER**
TROPFENSPENDER
DISTRIBUTEUR DE GOUTTE

(30) Priority: 06.05.2017 EP 17169837; 22.12.2017 EP 17210114
(43) Date of publication of application: 11.03.2020
(73) Proprietor: Novaliq GmbH, 69120 Heidelberg (DE)
(72) Inventor: LÖSCHER, Frank, 69198 Schriesheim (DE); KEMP, Philip, Thatcham Berkshire RG19 4GB (GB); MAUDEN, Jörg Martin, 69121 Heidelberg (DE); HOPPMANN, Eike, 86296 Greifenberg (DE); LÖSSL, Veronika, 83026 Rosenheim (DE); BURKHARDT, Sybille, 80687 München (DE)
(74) Representative: Pharma Patents International AG
(86) International application number: PCT/EP2018/061313
(87) International publication number: WO 2018/206386

(56) References cited:
- US-A1- 2005 274 744
- US-A1- 2010 006 600

## Description

### FIELD

The present invention relates to a drop dispenser or dropper bottle suitable for the administration of liquid compositions, especially for the topical administration of ophthalmic compositions in a dropwise manner, preferably for topical administration of ophthalmic compositions comprising semifluorinated alkanes (SFAs). In a further aspect, the present invention relates to a kit comprising a drop dispenser at least partially filled with a liquid composition.

### BACKGROUND

Semifluorinated alkanes (SFAs) are linear or branched compounds composed of at least one non-fluorinated hydrocarbon segment and at least one perfluorinated hydrocarbon segment. Semi-fluorinated alkanes have been described for various applications, for example commercially for unfolding and reapplying a retina, for long-term tamponade as vitreous humour substitute (H. Meinert et al., European Journal of Ophthalmology, Vol. 10(3), pp. 189-197, 2000), and as wash-out solutions for residual silicon oil after vitreo-retinal surgery.

WO2014/041055 describes mixtures of semifluorinated alkanes of the formula CF₃(CF₂)ₙ(CH₂)ₘCH₃. These mixtures are described to be ophthalmically applicable as tear film substitutes or for treating patients with dry eye syndrome and/or meibomian gland dysfunction.

It is known that the volume of drug instilled into the eye is of particular importance as it is one of the sources of drug response variation (German E.J. et.al, Eye 1999, 93-100).

Conventional eye drops are usually water-based compositions. When administering such water-based eye drops to the eye, the patient usually inverts the (eye-)dropper bottle that holds the ophthalmic composition and exerts a pressuring force to the flexible bottle in order to force one or more drops to be released from the (eye-)dropper bottle. This is usually done by simply squeezing the inverted eyedropper bottle resulting in the release of one or more drops (the aforementioned method is referred to as "pressure method" throughout this document).

Said conventional administration method (pressure method) known from water-based ophthalmic compositions is not suitable or not reliably suitable for administering ophthalmic compositions comprising SFAs, since SFA-comprising drops may be released from the eyedropper in a rather uncontrolled manner. Without being bound by theory, this is attributed to the interplay of the special surface properties of the amphiphilic SFAs, namely the interplay of high spreading capabilities, high density and/or low surface tension.

Furthermore, also ocular administration that relies only on the inversion of the (eye-) dropper bottle without exerting a pressuring force to the bottle (the aforementioned method is referred to as "inversion method" throughout this document) is not suitable or not reliably suitable for administering ophthalmic compositions comprising SFAs, since SFA-comprising drops are also released from the eyedropper in a highly uncontrolled manner employing said inversion method. Again, this is attributed to the interplay of the special surface properties of the amphiphilic SFAs, namely the interplay of high spreading capabilities, high density and/or low surface tension.

Thus, it is an object of the present invention to provide a drop dispenser or dropper bottle that allows for the reliable and controlled administration of liquid ophthalmic compositions, preferably for the reliable and controlled topical administration of compositions comprising semifluorinated alkanes (SFAs) to the eye of a patient in a drop-by-drop manner. US 2010/006600 A1 discloses an apparatus for metering and dispensing fluid includes a fluid dispensing section having an exit orifice, a fluid inlet, and a pathway extending from the fluid inlet to the exit orifice. A portion of the pathway includes a hydrophilic portion for metering a volume of the fluid when the dispensing section is upright; and a hydrophobic ring at the fluid inlet for preventing a metered volume from flowing out of the hydrophilic portion when the dispensing section is upright. The apparatus further includes a one-way valve between the exit orifice and the hydrophilic portion for allowing fluid to flow out of the hydrophilic portion and toward the exit orifice when sufficient pressure is applied to the fluid inlet. The one-way valve also prevents fluid from flowing through the exit orifice when the hydrophilic portion is being filled with fluid.

### SUMMARY OF THE INVENTION

In a first aspect, the invention relates to a drop dispenser (1), comprising:
- a container part (1B) with an interior volume adapted to be partially filled with a liquid phase (2) and a gaseous phase (3) filling the remainder of the interior volume at ambient pressure, the container part (1B) having a displaceable section (1C), and
- a dropper part (1A) in physical connection and in fluid communication with the interior volume of the container part (1B), comprising an outflow channel (5), connecting the interior volume of the container part (1B) to the environment;
   wherein the outflow channel (5) comprises
- a first section (5a) having a proximal end (5ap) and a distal end (5ad), each end having the same or different inner diameter selected independently from each other in the range of 0.09 to 0.19 mm; and
- a second section (5b) having a proximal end (5bp) and a distal end (5bd); the proximal end (5bp) having an inner diameter in the range of 1 to 3 mm; the distal end (5bd) having an inner diameter in the range of 0.1 to 3 mm, with the proviso that the inner diameter at the distal end of the second section (5bd) is larger than the inner diameter at the proximal end of the first section (5ap);
   and wherein
- the first section (5a) of the outflow channel (5) is generated by laser drilling.

In a second aspect, the invention relates to a kit comprising
- a drop dispenser (1) according to the first aspect of the invention at least partially filled with a liquid phase (2) and a gaseous phase (3), and
- directions for use of the drop dispenser (1).

In a third aspect, the present invention relates to a method or process for the manufacture of a drop dispenser (1) according to the first aspect of the invention or according to any of items 1 to 50, comprising the step of laser drilling the first section (5a) of the outflow channel.

Surprisingly, it was found that the drop dispenser of the present invention allows for the reliable, controlled and reproducible administration of liquid compositions, preferably topical administration of liquid compositions comprising semifluorinated alkanes (SFAs) to the eye of a patient in a dropwise manner. Furthermore, it was surprisingly found that the drop dispensers of the present invention allow for the convenient, reliable and reproducible administration of liquid ophthalmic compositions comprising a semifluorinated alkane in therapeutically relevant drop volumes in the range of 8 to 15 µL. Most importantly, the undesired spontaneous formation of drops or droplets can be reduced to a large extent or even completely avoided by using the drop dispensers of the present invention. This allows for precise and reliable dosing of the liquid compositions which is especially important for therapeutic applications. Furthermore, it allows for the maximum reduction of the amount of liquid composition that is lost or has to be discarded due to spontaneous and uncontrolled release from the drop dispenser.

Even further, the inventors found that the drop dispenser according to the first aspect of the present invention, works reliable also below ambient temperature, namely with ophthalmic compositions that were stored below ambient temperature (e.g. refrigerated compositions), which is usually problematic especially in the case of the dropwise administration of SFA-based compositions. Employing the drop dispenser of the present invention, said compositions may be directly administered without the need to equilibrate the composition to ambient temperature before use. The drop dispenser according to the first aspect of the present invention also works regardless of the volume of the headspace (gaseous volume that fills the remainder of the interior volume of the dropper bottle in addition to the liquid (ophthalmic) composition) in the dropper bottle. During ongoing use of the drop dispenser by dispensing the composition, the volume of the headspace in the drop dispenser is continuously increasing, as the volume of the liquid ophthalmic composition is decreasing. Such increasing headspace volume often hampers reliable administration of ophthalmic compositions utilizing conventional drop dispensers, especially when SFA-based compositions are employed - which, however is not observed when the drop dispenser according the present invention is employed.

### BRIEF DESCRIPTION OF THE DRAWINGS

Fig. 1: Schematic representation of a conventional drop dispenser (dropper bottle) (1) in the upright position
Fig. 2(A) and (B): Schematic representation of a drop dispenser (1) comprising a container part (bottle part) (1B) and a dropper part (1A)
Fig. 3: Schematic representation of a dropper part (1A) of a drop dispenser (1) according to the present invention
Fig. 4: Graphic representation of the results of Example 4

### DETAILED DESCRIPTION OF THE INVENTION

The terms "consist of", "consists of" and "consisting of" as used herein are so-called closed language meaning that only the mentioned components are present. The terms "comprise", "comprises" and "comprising" as used herein are so-called open language, meaning that one or more further components may or may not also be present.

The term "active pharmaceutical ingredient" (also referred to as "API" throughout this document) refers to any type of pharmaceutically active compound or derivative that is useful in the prevention, diagnosis, stabilization, treatment, or -generally speaking-management of a condition, disorder or disease.

The term "therapeutically effective amount" as used herein refers to a dose, concentration or strength which is useful for producing a desired pharmacological effect.

The term "dropwise" as used herein means that a liquid phase, more specifically a liquid composition is provided in a drop-by-drop fashion, which means that one discrete drop, irrespective of its size or volume, is provided or administered at a time and/or that a plurality of drops or droplets, preferably of the liquid composition, is provided in a consecutive manner, one at a time.

Further, according to the present invention, dropwise administration of the liquid composition is performed topically, meaning on the surface, e.g. to the skin or other outer boundary of a human or animal body or any part thereof. Preferably, the liquid composition is topically administered to the eye surface or an eye tissue.

The term "liquid composition" according to the present invention means any water-containing or water-free liquid, solution, emulsion or dispersion, preferably a liquid solution that may be applied to the human or animal body and that may optionally contain one or more active pharmaceutical ingredient (API) as defined above or further compounds like excipients, may optionally contain one or more active pharmaceutical ingredient (API) as defined above or further compounds like excipients, such as organic solvents, lipids, oils, lipophilic vitamins, lubricants, viscosity agents, acids, bases, antioxidants, stabilizers, synergists, coloring agents, thickening agents, - and if required in a particular cases - a preservative or a surfactant and mixtures thereof.

The term "distal" as used herein in connection with an end of the outflow channel (5) of the dropper part (1A) or an end of a section (5a) or (5b) of the outflow channel, respectively, is to be understood as being situated away or in further distance from the dropper mouth (7) or the location where the drops are dispensed from the dropper bottle as compared to the corresponding "proximal" end being situated closer to the dropper mouth (7) of the drop dispenser (1).

According to the first aspect, the present invention provides a drop dispenser (1), comprising:
- a container part (1B) with an interior volume adapted to be partially filled with a liquid phase (2) and a gaseous phase (3) filling the remainder of the interior volume at ambient pressure, the container part (1B) having a displaceable section (1C), and
- a dropper part (1A) in physical connection and in fluid communication with the interior volume of the container part (1B), comprising an outflow channel (5), connecting the interior volume of the container part (1B) to the environment;
   wherein the outflow channel (5) comprises
- a first section (5a) having a proximal end (5ap) and a distal end (5ad), each end having the same or different inner diameter selected independently from each other in the range of 0.09 to 0.19 mm; and
- a second section (5b) having a proximal end (5bp) and a distal end (5bd); the proximal end (5bp) having an inner diameter in the range of 1 to 3 mm; the distal end (5bd) having an inner diameter in the range of 0.1 to 3 mm, with the proviso that the inner diameter at the distal end of the second section (5bd) is larger than the inner diameter at the proximal end of the first section (5ap);
   and wherein
- the first section (5a) of the outflow channel (5) is generated by laser drilling.

In this first aspect of the present invention, a drop dispenser (1) is provided. A "drop dispenser" or "dropper bottle" as used herein synonymously may be a container, dispenser, applicator or bottle of any suitable kind for handheld use which can hold at least a single dose, preferably multiple doses of a liquid phase or composition and which may be designed of a single piece or multiple pieces or parts and which may typically be made of a material which is essentially inert against the liquid phase or composition to be administered. A drop dispenser (1) according to the present invention may be useful as a medical device that may be used as an eye drop delivery system (eyedropper), but which may also be helpful in administering certain compositions in a drop-by-drop manner to other parts of the body that are accessible to topical administration, such as ear, skin, nose, head, finger or other limbs. and which may be made of a rigid material, such as glass, (especially when used in a combination with a flexible material) or may be preferably made of a flexible material, such as, for example polyethylene or polypropylene. In a preferred embodiment of the present invention, the container part (1B) of the drop dispenser (1) is at least partially made of a flexible polymer, preferably of a flexible thermoplastic polymer.

The drop dispenser according to present invention comprises a container part (1B) and a dropper part (1A). The "container part" (1B) is the portion of the drop dispenser which holds the liquid phase (2) or composition to be administered dropwise in the amount of a single dose, preferably however in an amount of multiple doses or drops, typically in an amount of 0.1 to 15 ml, more typically in an amount of 0.1 to 10 ml, even more typically in an amount of 0.1 to 5 ml, especially when provided as multiple doses. The container part (1B) holds an interior space or volume which is at least partially filled with the liquid composition (2) to be administered. The container part (1B) also holds a gaseous phase (3) which fills the remainder of the interior volume which is not filled with the liquid composition (2) at ambient pressure. The gaseous phase (3) may consist of air or a protective gas or a mixture of air and a protective gas or a mixture of different protective gases and evaporated portions or traces of the components of the liquid composition (2). The gaseous phase (3) as well as the liquid composition (2) is held under ambient pressure, which means that it is under the same pressure as the surrounding atmospheric pressure, at least after the container has been opened.

The container part (1B) of the drop dispenser according to the present invention has a displaceable section (1C) and optionally a substantially stationary section. The term "displaceable section" as used herein may be any portion or area of the container part (1B) that may be displaced out of its original position relative to a fixed portion of the drop dispenser, e.g. relative to the dropper part (1A) of the drop dispenser by an external force applied, for example by pressing, pushing, shifting, tilting or bending out of its original position to a displaced position without affecting the physical integrity of the container part (1B). Preferably, the displacement of the displaceable section (1C) of the container part (1B) induces a deformation, preferably a reversible deformation of the container part, by which the inner volume of the container part is reduced. Optionally, the container part (1B) may also comprise a stationary or substantially stationary section which is not or substantially not displaced together with the displaceable section when an external force is applied. The stationary section may or may not be present and may be a separate part connected to the displaceable section (1C) or may be a portion of the displaceable section which may not be displaced relative to fixed portion of the drop dispenser.

The term "deformable wall part" (1C) as used herein synonymously to the term "displaceable section" in connection with the drop dispenser (1) of the present invention refers to the wall part of the container part (1B) of the drop dispenser (1), which is fabricated in such a way that allows to be deformed by a pressuring force exerted to it. The deformation of the wall part (1C) effects the interior volume of the container part to be compressed, resulting in the release of the gaseous phase (3) and/or the liquid (ophthalmic) composition (2) from the interior volume to the environment, as well as the intake of a gaseous phase (e.g. air) into the interior volume. The deformable wall part is preferably manufactured from a at least partially deformable material, preferably from an at least partially deformable plastic material, such as polypropylene or polyethylene. More preferably, the deformable wall part (1C) is manufactured from an at least partially manually deformable plastic material. Preferably, the deformable wall part (1C) is manufactured from an at least partially manually deformable plastic material with a preferred thickness in the range of from 0.4 to 1.6 mm, preferably with a thickness in the range of from 0.5 to 1.0 mm, more preferably with a thickness in the range of from 0.6 to 0.8 mm.

In preferred embodiments of the present invention, the dropper part (1A) and the container part (1B) are manufactured from plastic (polymeric) material, preferably from a thermoplastic polymeric material, such as, for example polypropylene and/or polyethylene. In particularly preferred embodiments, the dropper part (1A) is manufactured from polyethylene. It should be understood, however, that the container part (1B) and the dropper part (1A) may be prepared from the same or different polymeric materials as described above. In particularly preferred embodiments, however, the dropper part (1A) is manufactured from polyethylene and the container part (1B) of the drop dispenser (1) is manufactured from polypropylene.

The drop dispenser (1) provided according to the first aspect of the present invention also has a dropper part (1A). The "dropper part" is the portion of the drop dispenser through which the liquid composition (2) is discharged or physically released in a dropwise, i.e. in a drop-by-drop manner from the container part (1B) and subsequently administered. It may be physically connected to or, more specifically, mounted onto to the container part (1B) and connects the interior volume of the container part (1B) to the environment through an outflow channel (5) through which the liquid composition (2) to be administered is discharged.

The interior volume of the container part (1B), the first section (5a) and the second section (5b) are preferably in fluid communication. Said fluid communication allows both the liquid ophthalmic composition (2), as well as the gaseous phase (3) to be released from the dropper bottle (1) to the environment.

The term "container part" or "bottle part" (1B) of the dropper bottle (1), as used herein synonymously, refers to part of the dropper bottle (1) that holds the liquid (ophthalmic) composition (2) to be administered in its interior volume. Besides the liquid (ophthalmic) composition (2) the remaining part of the interior volume is filled by a gaseous phase (3). As described above, said gaseous phase (3) may comprise air or another gas, such as an inert gas (e.g. argon, nitrogen) or evaporated portions or traces of the components of the liquid composition (2). It is understood that, as the volume of the liquid composition (2) is decreasing upon repeated use/release of drops, the volume of the gaseous phase (3) is correspondingly increasing.

The term "outflow channel (5)" as used herein refers to a channel-like device or structure that connects the interior volume of the dropper bottle (1) to the environment, safeguarding the fluid (or gaseous) communication between interior volume and the environment. Herein, the outflow channel (5) is delimited at its distal end by the duct opening (4) located inside the interior volume of the dropper bottle and by the dropper mouth (7) at its proximal end located outside the interior volume of the dropper bottle (1). The outflow channel (5) has a total length extending from its distal end (4) to its proximal end (7). Usually, the inner diameter at the distal end (4) of the outflow channel (5) is smaller than the inner diameter at the proximal end (7) of the outflow channel. Upon administration, the liquid phase or composition, preferably the liquid ophthalmic composition is delivered from interior volume through the outflow channel (5) to the dropper mouth at the proximal end (7), where the composition is released in a drop-by-drop manner.

The outflow channel (5) of the drop dispenser according to the present invention comprises a first section (5a) having a proximal end (5ap) and a distal end (5ad), each end having the same or different inner diameter selected independently from each other in the range of 0.09 to 0.19 mm, preferably in the range of from about 0.10 to about 0.18 mm, more preferably from about 0.12 to about 0.18 mm, yet more preferably from about 0.12 to about 0.16 mm or even from about 0.14 to about 0.16 mm and most preferably of about 0.15 mm.

In a preferred embodiment, the first section (5a) of the outflow channel is located at the distal end (4) of the outflow channel. In a further preferred embodiment, the distal end (5ad) of the first section of the outflow channel (5) corresponds to the distal end of the outflow channel (the duct opening (4)). As described above, the term "duct opening (4)" as used herein refers to the distal end of the outflow channel (5). The duct opening (4) has preferably a circular cross-sectional shape and/or has a preferred diameter which may also be in the range of from 0.09 to 0.19 mm or the preferred ranges as described above for the distal end (5ad) and the proximal end (5ap) of the first section (5a) of the outflow channel.

As outlined above, both, the proximal end (5ap) and the distal end (5ad) of the first section (5a) of the outflow channel, independently from each other, may have an inner diameter in the range of 0.09 to 0.19 mm. This means that the inner diameters of the distal end (5ad) and the proximal end (5ap) of the first section (5a) of outflow channel (5) may be the same or different. In preferred embodiments, however, the inner diameter of the distal end (5ad) and the proximal end (5ap) of the first section (5a) of the outflow channel are (essentially) the same. Furthermore, in specific embodiments, the inner diameter of the outflow channel (5) is constant over the whole length of the first section (5a) of the outflow channel (5) from its distal end (5ad) to its proximal end (5ap). Accordingly, in some embodiments the first section (5a) of the outflow channel has a tubular shape with a constant inner diameter in the range of 0.09 to 0.19 mm.

In other embodiments, however, the first section (5a) of the outflow channel (5) may have varying inner diameters in the range of 0.09 to 0.19 mm or the preferred ranges as outlined above for the inner diameters of the proximal and (5ap) and the distal end (5ad) of the outflow channel resulting in, for example, irregularly shaped channel designs or channel designs of the first section (5a) of the outflow channel that have a substantially conical shape. In another preferred embodiment, the inner diameter of the first section (5a) of the outflow channel increases or decreases linearly from its proximal end (5ap) to its distal end (5ad). Preferably, the inner diameter at the distal end (5ad) is in the range of from about 0.16 to 0.19 mm and the inner diameter at the proximal end (5ap) is in the range of from about 0.09 to 0.15 mm, wherein the inner diameter gradually decreases from the distal end (5ad) to the proximal end (5ap). In another preferred embodiment, the inner diameter at the proximal end (5ap) is in the range of from 0.09 to 0.15 mm.

In addition to the first section (5a), the outflow channel (5) further comprises a second section (5b) having a proximal end (5bp) and a distal end (5bd). The proximal end (5bp) of the second section (5b) of the outflow channel has an inner diameter in the range of 1 to 3 mm. In some embodiments, the proximal end (5bp) of the outflow channel comprises or corresponds to the proximal end (7) of the outflow channel (5) (i.e. the dropper mouth) where the drops to be released from the drop dispenser are formed. In a preferred embodiment, the proximal end of the outflow channel (7) corresponds to the proximal end of the second section (5bp). The diameter of the proximal end (5bp) of the second section of the outflow channel preferably has a circular cross-sectional area. The diameter at the proximal end (5bp) is in the range of 1 to 3 mm, preferably in the range of 2 to 3 mm, more preferably in the range of 2.0 to 2.6 mm, even more preferably its diameter is in the range of about 2.0 to 2.4 mm. In a preferred embodiment, the proximal end of the outflow channel (7) corresponds to the proximal end (5bp) and its diameter is in the range of 1 to 3 mm, preferably in the range of 2 to 3 mm, more preferably in the range of 2.0 to 2.6 mm, even more preferably its diameter is in the range of about 2.0 to 2.4 mm. In another preferred embodiment, the inner diameter at the proximal end (7) of the outflow channel is in the range of about 2 to 3 mm, preferably of about 2.0. to 2.4 mm. The proximal end of the outflow channel (7) may have a circular cross-sectional area.

The second section (5b) of the outflow channel (5) also has a distal end (5bd) which has an inner diameter in the range of 0.1 to 3 mm, preferably 0.1 to 2.6 mm, more preferably 0.1 to 2.4 mm, or in the range of 0.2 to 3.0 mm, preferably 0.4 to 2.8 mm, more preferably 0.6 to 2.6 mm, or even more preferably 0.8 mm to 2.6 mm and most preferably in the range of 1.0 to 2.4 mm, with the proviso that the inner diameter at the distal end of the second section (5bd) is larger than the diameter at the proximal end of the first section (5ap). Accordingly, the inner diameter of the distal end (5bd) of the second section (5b) of the outflow channel (5) depends on the chosen inner diameter of the outflow channel at proximal end of the first section (5ap) which may be chosen in the range of 0.09 to 0.19 mm as outlined above. For example, in case the inner diameter of the outflow channel at the proximal end of the first section (5ap) is 0.19 mm, then the inner diameter at the distal end of the second section (5bd) may be chosen in the range starting from a value larger than 0.19 to 3 mm.

The inner diameter of the distal end of the second section (5bd) and the proximal end (5bp) of the second section (5b) of the outflow channel (5) may be the same or different from each other.

The second section (5b), as well as the first section (5a) of the outflow channel (5) independently from each other may have different cross-sectional shapes, such as circular, elliptic, rectangular or quadratic or the like, however, it is preferred that the second section (5b), as well as the first section (5a) of the outflow channel (5) have a circular cross-sectional area, wherein the term "cross-sectional area" is to be understood as the area perpendicular to the main longitudinal axis of the outflow channel (5). In cases, however, in which the sections of the outflow channel do not have a circular cross-sectional shape, but, for example an elliptic cross-sectional shape, the term "inner diameter" is to be understood as the largest diameter of such particular shape.

Accordingly, in case the inner diameters of the distal end (5bd) and the proximal end (5bp) of the second section of the outflow channel are the same and both have a substantially circular cross-sectional shape, the second section (5b) of the outflow channel may have a substantially tubular shape.

In other embodiments, however, the inner diameters of the distal end (5bd) and the proximal end (5bp) of the second section (5b) of the outflow channel (5) are different. In preferred embodiments, the inner diameter of the proximal end (5bp) of the second section of the outflow channel is larger than the inner diameter of the distal end (5bd) of the second section of the outflow channel. For example, in some embodiments the inner diameter of the second section (5b) of the flow channel increases linearly from the distal end (5bd) of the second section to the proximal end (5bp) of the second section.

In cases in which the second section (5b) of the outflow channel has a substantially circular cross-sectional area over its entire length and the inner diameter of the second section (5b) of the flow channel increases linearly from the distal end (5bd) of the second section to the proximal end (5bp) of the second section, the second section (5b) of the outflow channel has a substantially conical shape. However, other shapes of the second section (5b) may also be implemented, such as for example irregular shapes with varying inner diameters or inverted funnel shapes.

In a preferred embodiment, the distal end (5bd) of the second section (5b) of the outflow channel (5) is adjacent to the proximal end (5ap) of the first section (5a) of the outflow channel, or in other words, the proximal end (5ap) of the first section of the outflow channel is adjacent to the distal end (5bd) of the second section of the outflow channel. In this preferred embodiment, the second section (5b) is in direct contact with the first section (5a) of the outflow channel.

As outlined above, the outflow channel (5) comprises a first section (5a) and a second section (5b) and may, in further embodiments, optionally comprise further sections. In preferred embodiments, however, the outflow channel (5) comprises only said first section (5a) and said second section (5b), or in other words consists of said first section (5a) and said second section (5b). In these cases, the sum of the length of the first section (5a) and the length of the second section (5b) is equal to the overall length of the outflow channel (5).

In some preferred embodiments, the first section (5a) of the outflow channel (5) has a length of up to 30% or up to 25% of the total length of the outflow channel (5). Preferably, the first section of the outflow channel (5a) has a length of up to 25% of the total length of the outflow channel (5). In other embodiments, the first section (5a) of the outflow channel (5) may have a length of up to 20% or up to 10% of the total length of the outflow channel (5).

Accordingly, in some embodiments, the second section (5b) of the outflow channel (5) has a length of at least 70%, or of at least 75%, or of at least 80%, or of at least 90%, of the overall length of the outflow channel (5). Preferably, the second section of the outflow channel (5b) has a length of at least 75 % of the overall length of the outflow channel.

In some embodiments, the first section (5a) of the outflow channel (5) of the present drop dispenser may have a length of up to 4 mm, preferably a length of up to 3 mm, more preferably of up to 2.5 mm. The first section (5a) of the outflow channel (5) of the drop dispenser preferably has a length in the range of 0.5 to 4 mm, more preferably in the range of 0.5 to 3 mm, most preferably in the range of 0.5 to 2.5 mm, as measured from its distal end (5ad) to its proximal end (5ap). In further embodiments, the outflow channel (5) has an overall length of up to 15 mm, preferably has an overall length in the range of 7 to 13 mm, more preferably in the range of from about 7 to 12 mm. Accordingly, especially in cases in which the outflow channel only comprises said first section (5a) and said second section (5b), the second section (5b) of the outflow channel (5) of the present drop dispenser (1) has a length of up to 11 mm, preferably of up to 10 mm, more preferably of up to 9 mm. In a preferred embodiment, the second section of the outflow channel (5b) has a length in the range of 6.5 to 9.5 mm as measured from its distal end (5bd) to its proximal end (5bp).

According to the first aspect of the invention, the drop dispenser comprises an outflow channel (5) as described above with a first section (5a) and a second section (5b), wherein the first section (5a) of the outflow channel (5) having an inner diameter in the range of 0.09 to 0.19 mm is generated by laser drilling. According to the present invention, in general, it is possible that both sections, the first section (5a) as well as the second section (5b) may be produced or generated by laser drilling. In preferred embodiments, however, only the first section (5a) of the outflow channel (and not the second section (5b)) is produced or generated by laser drilling. In these embodiments, the second section (5b) may be prepared or generated or manufactured by standard manufacturing methods or techniques such as, for example, conventional drilling, moulding, or injection moulding, preferably together with injection moulding of the dropper part (1A) of the present drop dispenser (1).

The term "laser drilling" as used herein shall be understood broadly as any kind of drilling process or technique in which a laser or a laser beam is used to generate a hole or a channel like structure suitable as an outflow channel (5) in the drop dispenser (1) or, more specifically, in the dropper part (1A) of the drop dispenser according to the present invention. The laser drilling may be performed by using an UV-laser, an IR-laser or a CO₂ laser. Preferably the laser drilling is performed by using a UV-laser emitting in the range of 150 nm to 400 nm. A preferred UV-laser is one emitting at a wavelength in the range of 300 to 400 nm or in the range of 320 to 360 nm.

In a preferred embodiment, the laser drilling (also known as laser beam drilling) is performed by percussion laser beam drilling, wherein the laser beam 'punches' directly through the first section (5a) of the dropper part (1A) material with no relative movement of the laser or dropper part (1A). In preferred embodiments of the laser drilling process or, more specifically, percussion laser beam drilling process, an ultra-short pulse UV-laser with a pulse width of less than 50 picoseconds, preferably with a pulse width of less than 30 picoseconds or more preferably with a pulse width of less than 20 picoseconds, or with a pulse width in the range of from about 3 to about 20 picoseconds is utilized with a repetition rate in the range of about 50 kHz (kilohertz) to about 1000 kHz, preferably at a repetition rate of about 200 kHz to about 900 kHz, more preferably at a repetition rate of about 400 to about 800 kHz. The combination of an ultra-short pulse width of about 3 to 50 picoseconds in combination with a high repetition rate of about 50 to 1000 kHz allows to minimize the thermal damage of the dropper part (1A), or, more specifically, minimizes the thermal damage of the first section (5a) of the dropper part (1A).

In further preferred embodiments, the laser drilling or, more specifically, the percussion laser beam drilling as described above, an ultra-short pulse UV-laser with a pulse width of less than about 50 picoseconds, preferably with a pulse width of less than about 30 picoseconds or more preferably with a pulse width of less than about 20 picoseconds, or with a pulse width of between about 3 and 20 picoseconds is utilized with a pulse energy of less than about 60 µJ (microjoule), preferably less than about 40 µJ, more preferably less than about 20 µJ or between about 5µJ and 60 µJ. The combination of an ultra-short pulse within the range of about 3 to 50 picoseconds in combination with a low pulse energy in the range of about 5 to 60 µJ allows to minimize the thermal damage of the dropper part (1A), or, more specifically minimizes the thermal damage of the first section (5a) of the dropper part (1A).

Preferably, the section of the outflow channel (5), such as the first section (5a), that is fabricated by laser drilling is characterized by a lower surface roughness, when compared to the surface produced by standard manufacturing methods or techniques such as, for example, conventional drilling, moulding, or injection moulding.

In further preferred embodiments, the first section (5a) of the outflow channel (5) is generated by laser drilling, preferably by percussion laser beam drilling as described above, and the inner diameter of the proximal end (5ap) of the first section (5a) of the outflow channel (5) is larger than the inner diameter of the distal end (5ad), preferably the inner diameter of the proximal end (5ap) is at least about 5 %, preferably at least about 10 %, more preferably at least about 25 % larger than the inner diameter of the distal end (5ad) of the outflow channel (5). In further preferred embodiments, the inner diameter of the first section (5a) of the outflow channel (5) at the proximal end (5ap) is larger than the diameter of the distal end (5ad) by an amount in the range of from about 5 to 25%, preferably of from about 5 to 20%, more preferably of from about 5 to 15 %.

In further preferred embodiments, especially in cases in which the first section (5a) of the outflow channel (5) is generated by laser drilling as described above, more specifically by percussion laser beam drilling, the distance from the distal end (5ad) to the proximal end (5ap) of the first section (5a) of the outflow channel (5), or in other words the length of the first section (5a) of the outflow channel (5), is in the range of from about 0.5 to about 4 mm, preferably from about 0.5 to about 3 mm, more preferably from about 0.6 to about 2.5 mm, even more preferably it is from about 0.8 to about 2.2 mm.

In further preferred embodiments, the dropper part (1A) of the drop dispenser (1) of the present invention is made of a polymeric material that can be drilled by laser drilling techniques and, even more preferred, that can also be used for injection moulding, such as for example polyethylene and/or polypropylene, preferably polyethylene.

In the broadest aspect, the drop dispenser (1) according to the present invention comprises a container part (1B) with an interior volume adapted to be at least partially filled with a liquid phase (2) and a gaseous phase (3) filling the remainder of the interior volume at ambient pressure. Furthermore, the present invention also relates to the drop dispenser (1) according to the present invention, wherein the interior volume of the container part (1B) is at least partially filled with a liquid phase (2) and a gaseous phase (3) filling the remainder of the interior volume at ambient pressure.

The liquid phase (2) as referred to above may, in preferred embodiments, be a liquid composition, preferably a liquid ophthalmic composition to be administered topically to the surface of an eye of the patient. Accordingly, in preferred embodiments, the drop dispenser (1) according to the present invention comprises a liquid composition as the liquid phase (2). In further specific embodiments, the liquid composition is a liquid ophthalmic composition.

Accordingly, in further embodiments, the present invention relates to a drop dispenser (1) for topical administration, preferably dropwise topical administration, of an ophthalmic composition to the eye or to the surface of the eye of the patient, whereas the term "ophthalmic" as used herein means that the liquid composition can be topically administered to the eye, to the eye surface or to an eye tissue of a human or an animal.

In further embodiments, the liquid phase (2) or composition, preferably the liquid ophthalmic composition comprises a semifluorinated alkane. In some embodiments liquid ophthalmic composition as referred to above may comprise one or more different semifluorinated alkanes, usually in an amount of 50% (w/w) or more, 70% (w/w) or more, 80% (w/w) or more, 90% (w/w) or more, 95% (w/w) or more, 98% (w/w) or more, or even 99% (w/w) or more of the semifluorinated alkane or the mixture of semifluorinated alkanes, wherein the term "w/w" means the weight of the specific semifluorinated alkane per weight of the final composition to be administered. In another embodiment, the liquid phase (2) or composition to be administered by the drop dispenser (1) according to the present invention essentially consists of a semifluorinated alkane or a mixture of semifluorinated alkanes.

The term "semifluorinated alkane" (also referred to as "SFA" throughout this document) refers to a linear or branched compound composed of at least one perfluorinated segment (F-segment) and at least one non-fluorinated hydrocarbon segment (H-segment). More preferably, the semifluorinated alkane is a linear or branched compound composed of one perfluorinated segment (F-segment) and one non-fluorinated hydrocarbon segment (H-segment). Preferably, said semifluorinated alkane is a compound that exists in a liquid state at least at one temperature within the temperature range of 4° to 40°C. In a preferred embodiment, the perfluorinated segment and/or the hydrocarbon segment of the said SFA optionally comprises or consists of a cyclic hydrocarbon segment. Said SFA may comprise within the hydrocarbon segment an unsaturated moiety.

Preferably, the F-segment of a linear or branched SFA comprises between 2 to 10 or 3 to 10 carbon atoms. It is also preferred that the H-segment comprises between 3 to 10 carbon atoms. It is particularly preferred that the F- and the H-segment comprise, but independently from one another, 2 to 10 or 3 to 10 carbon atoms. Preferably, each segment independently from another is having carbon atoms selected from the range of 2 to 10 or 3 to 10.

It is further preferred, that the F-segment of a linear or branched, preferably linear SFA comprises between 4 to 10 carbon atoms and/or that the H-segment comprises between 4 to 10 carbon atoms. It is particularly further preferred that the F- and the H-segment comprise, but independently from one another, 4 to 10 carbon atoms. Preferably, each segment is independently from another having carbon atoms selected from the range of 4 to 10.

According to another nomenclature, the linear semifluorinated alkanes may be referred to as FnHm, wherein F means the perfluorinated hydrocarbon segment, H means the non-fluorinated hydrocarbon segment and n, m is the number of carbon atoms of the respective segment. For example, F4H5 is used for 1-perfluorobutylpentane.

Preferably, linear SFAs that may be comprised by the liquid phases or compositions to be dispensed by the drop dispenser according to the present invention are selected from the group consisting of F4H4, F4H5, F4H6, F4H7, F4H8, F5H4, F5H5, F5H6, F5H7, F5H8, F6H2, F6H4, F6H6, F6H7, F6H8, F6H9, F6H10, F6H12, F8H8, F8H10, F8H12, F10H10, more preferably said linear SFA is selected from the group consisting of F4H4, F4H5, F4H6, F5H4, F5H5, F5H6, F5H7, F5H8, F6H2, F6H4, F6H6, F6H7, F6H8, F6H9, F6H10, F8H8, F8H10, F8H12, F10H10, even more preferably the linear SFA is selected from the group consisting of F4H4, F4H5, F4H6, F5H4, F5H5, F5H6, F5H7, F5H8, F6H4, F6H6, F6H7, F6H8, F6H9, F6H10, F8H8, F8H10, F8H12, F10H10, most preferably the linear SFA is selected from the group consisting of F4H4, F4H5, F4H6, F5H5, F5H6, F5H7, F5H8, F6H6, F6H7, F6H8, F6H9, F6H10, F8H8, F8H10, F8H12, F10H10. In a further preferred embodiment, the linear SFA is selected from the group consisting of F4H5, F4H6, F5H6, F5H7, F6H6, F6H7, F6H8. In an even further preferred embodiment the linear SFA is selected from F4H5 and F6H8. In a particularly preferred embodiment the semifluorinated alkane comprised by the liquid phase (2) or composition to be dispensed by the drop dispenser (1) according to the present invention is F6H8.

In further embodiments, the liquid compositions to be dispensed by the drop dispenser (1) according to the present invention may further comprise organic solvents including, but not limited to, glycerol, propylene glycol, polyethylene glycol, ethanol, acetone, ethyl acetate, isopropyl alcohol, pentylene glycol, liquid paraffin, triglyceride oils and hydrofluorocarbons.

Furthermore, the liquid compositions to be dispensed by the drop dispenser (1) according to the present invention may further comprise potentially useful lipids or oily excipients including, but not limited to, triglyceride oils (e.g. soybean oil, olive oil, sesame oil, cotton seed oil, castor oil, sweet almond oil), mineral oil (e.g. petrolatum and liquid paraffin), medium chain triglycerides (MCT), oily fatty acids, isopropyl myristate, oily fatty alcohols, esters of sorbitol and fatty acids, oily sucrose esters, oily cholesterol esters, oily wax esters, glycerophospholipids, sphingolipids, or any oily substance which is physiologically tolerated by the eye.

Potentially useful antioxidants include, but are not limited to, vitamin E or vitamin E derivatives, ascorbic acid, sulphites, hydrogen sulphites, gallic acid esters, butyl hydroxyanisole (BHA), butyl hydroxytoluene (BHT) or acetylcysteine.

Further, the liquid phase (2) or composition to be dispensed by the present drop dispenser (1) may comprise one or more excipients, such as an organic cosolvent, such as an oil selected from glyceride oils, liquid waxes, and liquid paraffin or mineral oil, or said liquid composition may comprise an organic solvent exhibiting a high degree of biocompatibility, such as glycerol, propylene glycol, polyethylene glycol or ethanol.

The liquid phase (2) or composition may optionally further comprise one or more pharmaceutical active ingredients (APIs) such as for example: prostaglandin analogs (e.g. latanoprost, unoprostone, travoprost, bimatoprost, tafluprost), β-blockers, (e.g. timolol, brimonidine), cabonic anhydrase inhibitors (e.g. acetazolamide, dorzolamide, methazolamide, brinzolamide), antihistamines (e.g. olopatadine, levocabastine), corticosteroids (e.g. loteprednol, prednisolone, dexamethasone), fluorquinolone antibiotics (e.g. moxifloxacin, gatifloxacin, ofloxacin, levofloxacin), aminoglycoside antibiotics (e.g. tobramycin), macrolide antibiotics (e.g. azithromycin), VEGF-inhibitors (e.g. ranibizumab, bevacizumab, aflibercept), macrolide immunsuppressants (e.g. cyclosporine, tacrolimus, sirolimus), NSAIDs (e.g. bromfenac, nepafenac, diclofenac, ketorolac).

The liquid composition to be dispensed by the drop dispenser of the present invention may also comprise water, dissolved salts, buffer solutions and solvents known to those of skill in the art to be compatible with the above-described ophthalmic administration or may alternatively be a water-free composition.

One advantage of the reduced diameter of the first section (5a) of the outflow channel of the drop dispenser (1) according to the present aspect of the invention is that the undesired spontaneous outflow or drop-formation of the liquid composition, preferably the liquid ophthalmic composition can be reduced significantly for aqueous as well as non-aqueous compositions. Especially in cases of non-aqueous compositions, especially for SFA-containing compositions this has been found to be particularly beneficial.

It has been found that for practical purposes an inner diameter in the range of 0.09 to 0.19 mm of at least a portion of the outflow channel (section (5a)) is preferable as it combines significantly reduced spontaneous outflow with acceptable forces necessary to press the liquid composition through the outflow channel (5). Particularly in view of a possible use of the drop dispenser (1) according to the present invention characterized by a portion of the outflow channel (5) having an inner diameter in the range of 0.09 to 0.19 mm for the administration of SFA-containing ophthalmic compositions the reliability and ease of use has been shown of considerable importance for e.g. elderly or disabled users.

Furthermore, it has been found that the drop dispenser according to the present aspect of the invention offers higher precision and reproducibility of the drop sizes and volumes to be dispensed, independent of the temperature of the drop dispenser, liquid composition and/or the environment as well as the actual filling level of the drop dispenser or "headspace" above the liquid composition in the drop dispenser.

In a second aspect, the present invention relates to a kit comprising
- a drop dispenser (1) according to the first aspect of the invention at least partially filled with a liquid phase (2) and a gaseous phase (3), and
- directions for use of the drop dispenser (1).

The kit according to this aspect of the invention comprises a drop dispenser (1) or dropper bottle as described in detail above in connection with the first aspect of the invention.

The drop dispenser (1) of the kit according to this aspect of the invention is at least partially filled with a liquid phase (2) and a gaseous phase (3). In preferred embodiments, the liquid phase is a liquid composition, preferably a liquid ophthalmic composition as described above in connection with first aspect of the invention. In specific embodiments, the liquid phase (2) or preferably the liquid composition to be dispensed by the drop dispenser (1) of the first aspect of the present invention comprises a semifluorinated alkane as described above in connection with the first aspect of the present invention.

In addition to the drop dispenser (1) according to the first aspect of the invention which is at least partially filled with a liquid phase (2) and a gaseous phase (3) filling the remaining volume of the container part (1B) of the drop dispenser, the kit of this second aspect of the invention also comprises directions for the use of the drop dispenser (1) according to the first aspect of the invention.

The directions or instructions for use comprised by the kit according to this aspect of the invention may be in in any form suited to instruct the user how to perform the topical or topical ophthalmic administration of a liquid phase or liquid composition, preferably comprising or even essentially consisting of semifluorinated alkane. It may be in any readable or tangible form, preferably in printed form or in any machine- or computer-readable form, preferably in form of a machine-readable optical label such as, for example, a barcode or a QR-code. In particularly preferred embodiments, the directions for use are provided in form of an instruction leaflet, product or package insert or as an enclosed label. Preferably the directions or instructions for use are provided in printed form, for example in form of a printed label, which may be provided together with the drop dispenser (1) or dropper bottle according to the first aspect of the invention. For example, such a label may be packaged together with the said drop dispenser (1) or dropper bottle.

In a third aspect, the present invention relates to a method or process for the manufacture or production of a drop dispenser (1) according to the first aspect of the invention comprising the step of laser drilling the first section (5a) of the outflow channel.

According to this aspect of the invention, the method or process also comprises the step of providing a precursor of the drop dispenser (1) according to the first aspect of the invention, or, more specifically, a precursor of the dropper part (1A) of the drop dispenser (1) of the present invention, which does not comprise the first section (5a) of the outflow channel yet, or in other words, in which the first section of the outflow channel has not yet been generated by either drilling, moulding or other suitable process. In this precursor, the second section (5b) of the outflow channel (5) may or may not be present. In preferred embodiments, however, the method of the present aspect of the invention comprises the step of providing a precursor of the drop dispenser (1) or the dropper part (1A) of the drop dispenser in which the second section (5b) of the outflow channel (5) is present or has already be formed, and further the step of generating the first section of the outflow channel by laser drilling as described above.

Accordingly, the present aspect of the invention also provides for a method or process for the manufacture or production of a drop dispenser (1) comprising the step of
a) providing a precursor of the drop dispenser (1) according to the first aspect of the invention or a precursor of the dropper part (1A) of the drop dispenser (1) of the present invention, which does not comprise the first section (5a) of the outflow channel; and
b) laser drilling the first section (5a) of the outflow channel.

For the avoidance of doubt, it should be pointed out that all features of the drop dispenser (1) or its various parts as described for the first aspect of the invention also apply to this third aspect of the invention.

### DESCRIPTION OF THE DRAWINGS

List of reference numerals:
- 1: drop dispenser or dropper bottle
- 1A: dropper part of the drop dispenser (1) or dropper bottle
- 1B: container part or bottle part of the drop dispenser (1) or dropper bottle
- 1C: displaceable section or deformable wall part of the container part (1B) of the drop dispenser (1)
- 2: liquid composition
- 3: gaseous phase
- 4: duct opening of the outflow channel (5)
- 5: outflow channel
- 5a: first section of the outflow channel (5)
- 5ad: distal end of the first section (5a) of the outflow channel
- 5ap: proximal end of the first section (5a) of the outflow channel
- 5b: second section of the outflow channel (5)
- 5bd: distal end of the second section (5b) of the outflow channel
- 5bp: proximal end of the second section (5b) of the outflow channel
- 7: dropper mouth of the outflow channel (5)

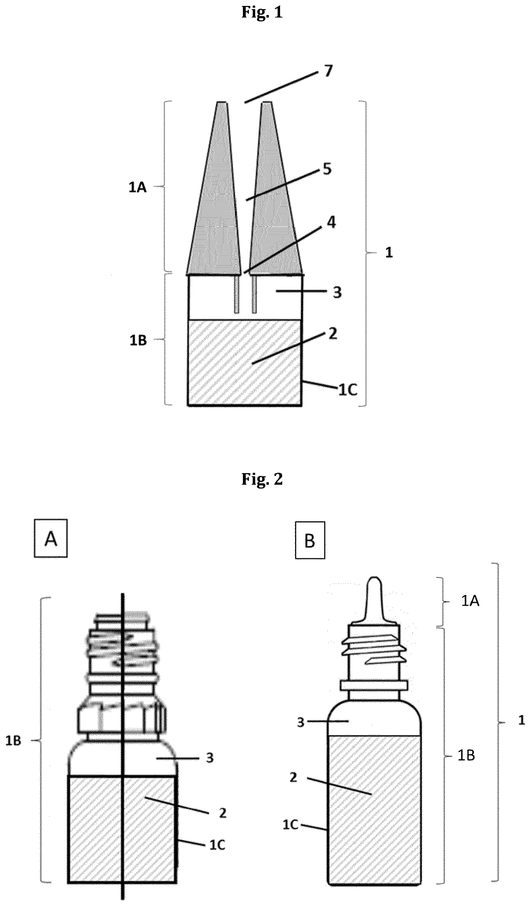

Fig. 1 shows a schematic representation of a conventional drop dispenser (1) or a dropper bottle in the upright position with the dropper mouth (7) of the outflow channel (5) facing upwards. The drop dispenser or dropper bottle (1) comprises a container or bottle part (1B) and a dropper part (1A). The container part or bottle part (1B) comprises an interior volume that is at least partially filled with a liquid ophthalmic composition (2). The remainder of the interior volume of the container or bottle part (1B) is filled with a gaseous phase (3). The wall of the container or bottle part has a displaceable section (1C) to allow a pressuring force to compress the interior volume (e.g. by manual squeezing). The dropper part (1A) is mounted onto the container or bottle part (1B), connecting the interior volume of the bottle part (1B) via the outflow channel (5) to the environment. Herein, the fluid communication of the interior volume of the container or bottle part (1B) to the environment is effected by the outflow channel (5) from distal to proximal end. The outflow channel (5) is delimited by the duct opening (4) at the distal end and by the dropper mouth (7) at the proximal end. In this upright position, the liquid composition (2) does not contact the outflow channel (5).

Fig. 2 shows a schematic representation of a dropper bottle (1) according to the present invention comprising a bottle part (1B) and a dropper part (1A).

In Fig. 2(A) an exemplary bottle part (1B) is shown, comprising an interior volume that is at least partially filled with the liquid ophthalmic composition (2) and a gaseous phase (3) filing the remainder of the interior volume of the bottle part (1B). The wall of the bottle part is deformable (1C) as to allow a pressuring force (e.g. manual squeezing) to compress the interior volume.

Fig. 2(B) shows an exemplary dropper bottle (1) comprising said dropper part (1A) mounted onto the bottle part (1B).

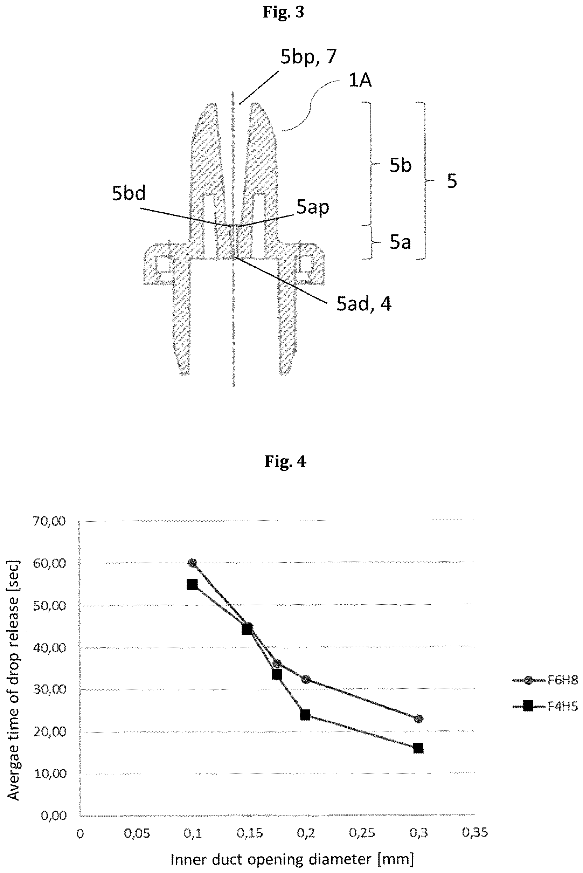

Fig. 3 shows another exemplary dropper part (1A) according to the present invention comprising an outflow channel (5) being delimited at its distal end by the duct opening (4) and by the dropper mouth (7) at its proximal end. In this particular embodiment, the outflow channel (5) has the two distinct sections (5a) and (5b) with the first section (5a) of the outflow channel (5) having a constant inner diameter from its distal end (5ad) to its proximal end (5ap). The inner diameter of the second section (5b) of the outflow channel (5), however, increases continuously from the distal end (5bd) towards the proximal end (5bp), thereby generating a substantially conical second section (5b) of the outflow channel. Further, in this particular embodiment, the proximal end (5bp) of the second section (5b) of the outflow channel corresponds to the dropper mouth (7) and the proximal end (5ap) of the first section of the outflow channel corresponds to the duct opening (4).

In this particular embodiment of the present invention, the first section (5a) of the outflow channel (5) has a constant inner diameter of 0.15 mm from the distal end (5ad) through the proximal end (5ap). The second section (5b) is adjacent to, i.e. in direct contact with the first section (5a) of the outflow channel (5) and has a larger inner diameter which is continuously increasing towards the dropper mouth (7) corresponding to the proximal end (5bp) of the second section (5b) outflow channel (5) to a diameter e.g. in the range of 2.0 to 2.4 mm.

Fig. 4 is a graphic representation of the results of Example 4 as outlined below and as summarized in Tables 6 to 11. The graphs show the relation between the average time of self-dropping observed in 60 seconds against the inner diameter of the distal end (5ad) of the first section (5a) of the outflow channel of a polyproylene drop dispenser (1) (duct opening diameter) of the drop dispenser.

The following examples serve to illustrate the present invention without, however, limiting it in any respect:

### EXAMPLES

### Example 1: NovaTears® ophthalmic composition

The liquid NovaTears® (Novaliq GmbH, Germany) ophthalmic composition for treating dry eye disease, comprises 1-Perfluorohexyloctane (F6H8) and is provided in a dropper bottle (1) with a polyethylene dropper part (1A) mounted to a polypropylene bottle part (1B) for holding 3 ml of NovaTears®. The dropper part (1A) comprises an outflow channel (5) with a dropper mouth (7) (diameter 2.4 mm) at its proximal end and a duct opening (4) (0.3 mm diameter) at its distal end. Upon inversion of the dropper bottle (1), the liquid ophthalmic composition flows from the interior volume to the proximal end of the outflow channel (5). Herein, the liquid enters into the outflow channel (5) at the duct opening (4) and continues to the dropper mouth (7), where it is released as a drop.

### Example 2: Comparative drop size analysis of polypropylene drop dispenser with different outflow channel diameters filled with F6H8 at different fill levels

Three polypropylene droppers (Packsys®) with a duct opening (4) diameter of 0.3 mm at the distal end of its outflow channel and a dropper mouth (7) diameter of 2.4 mm at the proximal end of its outflow channel ("Dropper 14182") were assembled on bottles filled with 1 ml, 3 ml and 5 ml of F6H8. Prior to testing, the bottles were closed with dropper and cap. The cap was removed and sample fluid F6H8 was dispensed dropwise. 5 drops of F6H8 were collected from the start, middle and end of each sample (5 ml; 3 ml; 1 ml respectively) were weighed and the corresponding drop sizes calculated on the basis of the density of F6H8 (1.331gcm⁻³). Table 1 shows the resulting average drop weights and volumes:

**Table 1:**

| ***Dropper 14182*** | F6H8 | | | |
|---|---|---|---|---|
| | 5mL | 3mL | 1mL | Average |
| Average Drop Weight (mg) | 14.967 | 15.458 | 15.035 | 15.153 |
| % RSD of Drop Weight | 0.940 | 0.873 | 1.061 | 0.964 |
| Average Drop Volume (µL) | 11.245 | 11.614 | 11.296 | 11.385 |
| % RSD of Drop Size | 0.706 | 0.656 | 0.797 | 0.725 |

The experiment was repeated using three polypropylene droppers (Packsys®) with a duct opening (4) diameter of 0.15 mm and a dropper mouth (7) diameter of 2.4 mm ("Dropper 14014"). Table 2 shows the resulting average drop weights and volumes:

**Table 2:**

| ***Dropper 14014*** | F6H8 | | | |
|---|---|---|---|---|
| | 5mL | 3mL | 1mL | Through-Life |
| Average Drop Weight (mg) | 14.509 | 14.540 | 14.498 | 14.516 |
| % RSD of Drop Weight | 0.646 | 0.760 | 0.699 | 0.687 |
| Average Drop Volume (µL) | 10.901 | 10.924 | 10.893 | 10.906 |
| % RSD of Drop Size | 0.486 | 0.571 | 0.525 | 0.516 |

Table 3 shows the drop weights in mg as measured for the three droppers, each having a duct opening (7) diameter of 0.3 mm and a dropper mouth (7) diameter of 2.4 mm (Dropper 14182).

**Table 3:**

| Fill Volume | 14182 | | |
|---|---|---|---|
| | F6H8 Drop Weight (mg) | | |
| | 1 | 2 | 3 |
| 5mL | 15.260 | 16.089 | 14.115 |
| | 13.397 | 15.257 | 13.251 |
| | 15.168 | 15.620 | 16.119 |
| | 14.873 | 14.525 | 16.155 |
| | 14.105 | 14.813 | 15.753 |
| 3mL | 15.693 | 14.928 | 17.034 |
| | 15.739 | 15.120 | 16.204 |
| | 14.668 | 14.648 | 16.424 |
| | 15.949 | 14.665 | 16.584 |
| | 15.058 | 13.855 | 15.295 |
| 1mL | 14.637 | 15.426 | 16.599 |
| | 14.877 | 13.054 | 16.255 |
| | 13.947 | 14.200 | 13.827 |
| | 15.543 | 14.526 | 16.084 |
| | 16.526 | 14.572 | 15.451 |

Table 4 shows the comparison of the drop sizes generated with droppers having a duct opening (4) of either 0.3 mm or 0.15 mm. As can be seen, a smaller diameter of the duct opening (4) helps to adjust the drop volume to a target drop volume of about 10 µl.

Furthermore, a smaller duct opening (4) allows to realize more constant drop volumes, independent from the fill level of the dropper bottle used.

**Table 4:**

| | Material: F6H8 | | | |
|---|---|---|---|---|
| | 5mL | 3mL | 1mL | Average |
| 14182 Average Drop Volume (µL) | 11.245 | 11.614 | 11.296 | 11.385 |
| 14182 % RSD of Drop Size | 0.706 | 0.656 | 0.797 | 0.725 |
| 14014 Average Drop Volume (µL) | 10.901 | 10.924 | 10.893 | 10.906 |
| 14014 % RSD of Drop Size | 0.486 | 0.571 | 0.525 | 0.516 |

Example 3: Testing of a polypropylene drop dispenser with a duct opening (4) diameter of 0.15 mm filled with F6H8 at different filling levels

In this experiment, three series of five polypropylene droppers with a duct opening (4) diameter of 0.15 mm and a dropper mouth (7) diameter of 2.4 mm ("Dropper 14014") were assembled on bottles having a total volume of 5 ml which were filled with F6H8 at different filling volumes: 5 droppers (droppers 1 to 5) were filled with 0,2 ml F6H8 each (filling level "nearly empty"); 5 droppers were filled with 3 ml F6H8 each (filling level "half full") and 5 droppers were filled with 5 ml F6H8 each (filling level "full") at room temperature. The dropper bottles were opened and inverted by 180° to a vertical orientation with the dropper mouth pointing downwards. For a period of 10 s it was observed whether the spontaneous formation of drops occurred. The drops, if formed, were counted. Table 5 summarizes the results of the experiment with "OK" depicting that no drops were formed within 10 s from the inversion of the bottle.

**Table 5:**

| Dropper | Filling volume 0,2 ml (nearly empty) | Filling volume 3 ml (half full) | Filling volume 5 ml (full) |
|---|---|---|---|
| 1 | OK | OK | OK |
| 2 | OK | OK | OK |
| 3 | OK | OK | OK |
| 4 | OK | OK | OK |
| 5 | OK | OK | OK |

Example 4: Testing of drop dispensers (1) comprising a polyethylene dropper part (1A) and a polypropylene container part (1B) filled with F6H8 or F4H5 at different filling levels

In this experiment four dropper parts (1A) having different outflow channel dimensions (duct diameters of 0.3 mm, 0.175 mm, 0.15 mm, 0.1 mm) were assembled on container parts (1B) having a total volume of 5 ml. These 4 drop dispensers were filled with F6H8 and F4H5, respectively, at room temperature at different filling volumes of 3.75 ml, 2.5 ml and 1.25 ml, respectively. At ambient pressure, the drop dispensers were inverted by 180° to a vertical orientation with the dropper mouth (7) pointing downwards. For a period of 60 s it was observed whether the spontaneous formation of drops occurred and after how many seconds. The following tables 6 to 11 summarize the results of the experiment. The experiment as described above were conducted three times and the results of each run summarized in columns 1 to 3, respectively. The values given denote the duration in seconds until the formation of a drop was observed after inversion of the respective drop dispensers:

**Table 6:**

| outflow channel (5) dimensions/mm | | | | F6H8 Filling volume 3.75 ml | | |
|---|---|---|---|---|---|---|
| 5ad | 5ap | 5bd | 5bp | 1 | 2 | 3 |
| 0.3 | 0.3 | 1.0 | 2.4 | 7, 14, 27, 49 s | 8, 14, 23, 36, 53 s | 12, 21, 33, 47 s |
| 0.175 | 0.175 | 1.0 | 2.4 | 27, 39 s | 23, 32, 44 s | 25, 33, 43, 56 s |
| 0.15 | 0.15 | 1.0 | 2.4 | 42, 55 s | 29, 39, 51 s | 32,46 s |
| 0.10 | 0.10 | 1.0 | 2.4 | None | None | None |

**Table 7:**

| outflow channel (5) dimensions/mm | | | | F6H8 Filling volume 2.5 ml | | |
|---|---|---|---|---|---|---|
| 5ad | 5ap | 5bd | 5bp | 1 | 2 | 3 |
| 0.3 | 0.3 | 1.0 | 2.4 | 4, 8, 15, 23, 46 s | 3, 5, 8, 14, 21, 30, 40, 54 s | 4, 6, 8,13, 20, 31, 44 s |
| 0.175 | 0.175 | 1.0 | 2.4 | 28,37,48 s | 19, 25, 32, 41, 53 s | 19, 24, 32, 42,55 s |
| 0.15 | 0.15 | 1.0 | 2.4 | 35,46, 57 s | 34, 44, 56 s | 50 s |
| 0.10 | 0.10 | 1.0 | 2.4 | None | None | None |

**Table 8:**

| outflow channel (5) dimensions/mm | | | | F6H8 Filling volume 1.25 ml | | |
|---|---|---|---|---|---|---|
| 5ad | 5ap | 5bd | 5bp | 1 | 2 | 3 |
| 0.3 | 0.3 | 1.0 | 2.4 | 4,6,8,15,26,39, 58s | 7,10,15,19,25,32, 41,52s | 4, 7, 11, 16, 23, 33,45 s |
| 0.175 | 0.175 | 1.0 | 2.4 | 22, 28, 35, 43, 53 s | 26, 34, 41, 50 s | 25, 34, 39, 46, 55 s |
| 0.15 | 0.15 | 1.0 | 2.4 | 47, 59 s | 28, 33, 40, 47, 55 s | 28, 34, 42, 50, 58 |
| 0.10 | 0.10 | 1.0 | 2.4 | None | None | None |

**Table 9:**

| outflow channel (5) dimensions/mm | | | | F4H5 Filling volume 3.75 ml | | |
|---|---|---|---|---|---|---|
| 5ad | 5ap | 5bd | 5bp | 1 | 2 | 3 |
| 0.3 | 0.3 | 1.0 | 2.4 | 6, 19,40 s | 2, 4, 6, 13, 23, 40 s | 3, 8, 20, 40 s |
| 0.175 | 0.175 | 1.0 | 2.4 | 47 s | 29, 59 s | 11, 17, 24, 36, 49, 58 s |
| 0.15 | 0.15 | 1.0 | 2.4 | 33 s | None | None |
| 0.10 | 0.10 | 1.0 | 2.4 | 49 s | 57 s | 43, 59 s |

**Table 10:**

| outflow channel (5) dimensions/mm | | | | F4H5 Filling volume 2.5 ml | | |
|---|---|---|---|---|---|---|
| 5ad | 5ap | 5bd | 5bp | 1 | 2 | 3 |
| 0.3 | 0.3 | 1.0 | 2.4 | 5, 10, 17, 31 s | 2, 4, 7, 9, 12, 16, 22, 30, 56 s | 2, 5, 12, 27 s |
| 0.175 | 0.175 | 1.0 | 2.4 | 27, 41 s | 12,19,32,52 s | 7, 9, 11, 15, 18, 23, 29,38, 50 s |
| 0.15 | 0.15 | 1.0 | 2.4 | 22, 55 s | 16, 24, 36 s | 49 s |
| 0.10 | 0.10 | 1.0 | 2.4 | 59 s | 53 s | None |

**Table 11:**

| outflow channel (5) dimensions/mm | | | | F4H5 Filling volume 1.25 ml | | |
|---|---|---|---|---|---|---|
| 5ad | 5ap | 5bd | 5bp | 1 | 2 | 3 |
| 0.3 | 0.3 | 1.0 | 2.4 | 4, 12,32 s | 2, 4, 5, 8, 13, 19, 28, 42 s | 3, 4, 7, 12, 22, 33 s |
| 0.175 | 0.175 | 1.0 | 2.4 | 41s | 27 s | 9, 10, 13, 16, 20, 26, 34, 50 s |
| 0.15 | 0.15 | 1.0 | 2.4 | 20,32 s | 41, 58 s | 59 s |
| 0.10 | 0.10 | 1.0 | 2.4 | None | None | 46 s |

The results as shown in Tables 6 to 11 have been further summarized and graphically represented as shown in Fig. 6. The graph shows the relation between the average time of self-dropping observed in 60 seconds against the duct diameter (4) (with inner diameters 4 = 5ap = 5ad) of the 4 different drop dispensers. The average time of self-dropping observed in 60 seconds is calculated from the data collected using drop dispensers differing in duct diameter (4) and assembled on container parts (1A) differing in filling volumes, as described in Example 4.

## Claims

1. A drop dispenser (1), comprising:
- a container part (1B) with an interior volume adapted to be partially filled with a liquid phase (2) and a gaseous phase (3) filling the remainder of the interior volume at ambient pressure, the container part (1B) having a displaceable section (1C), and
- a dropper part (1A) in physical connection and in fluid communication with the interior volume of the container part (1B), comprising an outflow channel (5), connecting the interior volume of the container part (1B) to the environment;
wherein the outflow channel (5) comprises
- a first section (5a) having a proximal end (5ap) and a distal end (5ad), each end having the same or different inner diameter selected independently from each other in the range of 0.09 to 0.19 mm; and
- a second section (5b) having a proximal end (5bp) and a distal end (5bd); the proximal end (5bp) having an inner diameter in the range of 1 to 3 mm; the distal end (5bd) having an inner diameter in the range of 0.1 to 3 mm, with the proviso that the inner diameter at the distal end of the second section (5bd) is larger than the inner diameter at the proximal end of the first section (5ap);
and wherein
- the first section (5a) of the outflow channel (5) is generated by laser drilling.

2. The drop dispenser according to claim 1, wherein the sum of the length of the first section (5a) and the length of the second section (5b) is equal to the overall length of the outflow channel (5).

3. The drop dispenser according to any preceding claim, wherein the first section (5a) of the outflow channel is located at the distal end (4) of the outflow channel.

4. The drop dispenser according to any preceding claim, wherein the distal end (5bd) of the second section of the outflow channel (5) is adjacent to the proximal end (5ap) of the first section (5a) of the outflow channel.

5. The drop dispenser according to any preceding claim, wherein the inner diameter of the proximal end (5ap) of the first section (5a) of the outflow channel (5) is larger than the diameter of the distal end (5ad) of the first section (5a) of the outflow channel (5).

6. The drop dispenser according to any preceding claim, wherein the inner diameter of the second section (5b) of the outflow channel increases linearly from the distal end (5bd) of the second section (5b) to the proximal end (5bp) of the second section.

7. The drop dispenser according to any preceding claim, wherein the first section (5a) and/or the second section (5b) of the outflow channel have a circular cross-sectional area.

8. The drop dispenser according to any of the preceding claims, wherein the inner diameter at the proximal end (7) of the outflow channel is in the range of about 2 to 3 mm.

9. The drop dispenser according to any of the preceding claims, wherein the drop dispenser (1) comprises a liquid composition as the liquid phase (2), wherein the liquid phase (2) comprises a semifluorinated alkane, preferably selected from F4H5 and F6H8.

10. The drop dispenser according to any of the preceding claims, wherein the dropper part (1A) is made of a polymeric material that can be drilled by laser drilling techniques and, used for injection moulding..

11. The drop dispenser according to any preceding claim, wherein the first section (5a) of the outflow channel (5) is generated by laser drilling using an UV-laser, an IR-laser or a CO₂ laser.

12. The drop dispenser of any preceding claim, wherein the second section (5b) of the outflow channel (5) is generated by conventional drilling, moulding or injection moulding.

13. The drop dispenser according to any preceding claim, wherein the inner diameter of the proximal end (5ap) of the first section (5a) of the outflow channel (5) is at least 5%, preferably at least 10%, more preferably at least 25 % larger than the inner diameter of the distal end (5ad) of the first section (5a) of the outflow channel (5).

14. A method or process for the manufacture of a drop dispenser (1) according to any of claims 1 to 13, comprising the step of laser drilling the first section (5a) of the outflow channel.

15. A kit comprising
- a drop dispenser (1) according to any of the claims 1 to 13 and
- directions for use of the drop dispenser (1).

## Patentansprüche

1. Tropfenabgabevorrichtung (1), die Folgendes umfasst:
- einen Behälterteil (1B) mit einem Innenvolumen, das ausgelegt ist, teilweise mit einer Flüssigphase (2) und einer Gasphase (3), die bei Umgebungsdruck den verbleibenden Teil des Innenvolumens füllt, gefüllt zu sein, wobei der Behälterteil (1B) einen verschiebbaren Abschnitt (1C) aufweist, und
- einen Tropfvorrichtungsteil (1A) in physischer Verbindung und in strömungstechnischer Kommunikation mit dem Innenvolumen des Behälterteils (1B), umfassend einen Abflusskanal (5), der das Innenvolumen des Behälterteils (1B) mit der Umgebung verbindet;
wobei der Abflusskanal (5) umfasst
- einen ersten Abschnitt (5a) mit einem proximalen Ende (5ap) und einem distalen Ende (5ad), wobei jedes Ende denselben oder einen unterschiedlichen Innendurchmesser aufweist, die unabhängig voneinander in dem Bereich von 0,09 bis 0,19 mm ausgewählt sind; und
- einen zweiten Abschnitt (5b) mit einem proximalen Ende (5bp) und einem distalen Ende (5bd); wobei das proximale Ende (5bp) einen Innendurchmesser in dem Bereich von 1 bis 3 mm aufweist; wobei das distale Ende (5bd) einen Innendurchmesser in dem Bereich von 0,1 bis 3 mm aufweist, in dem Sinne, dass der Innendurchmesser an dem distalen Ende des zweiten Abschnitts (5bd) größer als der Innendurchmesser an dem proximalen Ende des ersten Abschnitts (5ap) ist;
und wobei
- der erste Abschnitt (5a) des Abflusskanals (5) durch Laserbohren erzeugt ist.

2. Tropfenabgabevorrichtung nach Anspruch 1, wobei die Summe der Länge des ersten Abschnitts (5a) und der Länge des zweiten Abschnitts (5b) gleich der Gesamtlänge des Abflusskanals (5) ist.

3. Tropfenabgabevorrichtung nach einem der vorhergehenden Ansprüche, wobei der erste Abschnitt (5a) des Abflusskanals an dem distalen Ende (4) des Abflusskanals angeordnet ist.

4. Tropfenabgabevorrichtung nach einem der vorhergehenden Ansprüche, wobei das distale Ende (5bd) des zweiten Abschnitts des Abflusskanals (5) an das proximale Ende (5ap) des ersten Abschnitts (5a) des Abflusskanals angrenzt.

5. Tropfenabgabevorrichtung nach einem der vorhergehenden Ansprüche, wobei der Innendurchmesser des proximalen Endes (5ap) des ersten Abschnitts (5a) des Abflusskanals (5) größer als der Durchmesser des distalen Endes (5ad) des ersten Abschnitts (5a) des Abflusskanals (5) ist.

6. Tropfenabgabevorrichtung nach einem der vorhergehenden Ansprüche, wobei der Innendurchmesser des zweiten Abschnitts (5b) des Abflusskanals linear von dem distalen Ende (5bd) des zweiten Abschnitts (5b) zu dem proximalen Ende (5bp) des zweiten Abschnitts ansteigt.

7. Tropfenabgabevorrichtung nach einem der vorhergehenden Ansprüche, wobei der erste Abschnitt (5a) und/oder der zweite Abschnitt (5b) des Abflusskanals einen runden Querschnittsbereich aufweisen.

8. Tropfenabgabevorrichtung nach einem der vorhergehenden Ansprüche, wobei der Innendurchmesser an dem proximalen Ende (7) des Abflusskanals in dem Bereich von ca. 2 bis 3 mm ist.

9. Tropfenabgabevorrichtung nach einem der vorhergehenden Ansprüche, wobei die Tropfenabgabevorrichtung (1) eine flüssige Zusammensetzung als die Flüssigphase (2) umfasst, wobei die Flüssigphase (2) ein semifluoriertes Alkan, vorzugsweise ausgewählt aus F4H5 und F6H8, umfasst.

10. Tropfenabgabevorrichtung nach einem der vorhergehenden Ansprüche, wobei der Tropfvorrichtungsteil (1A) aus einem Polymermaterial besteht, das durch Laserbohrtechniken gebohrt werden kann und für Spritzgießen verwendet wird.

11. Tropfenabgabevorrichtung nach einem der vorhergehenden Ansprüche, wobei der erste Abschnitt (5a) des Abflusskanals (5) durch Laserbohren mithilfe eines UV-Lasers, eines IR-Lasers oder eines CO₂-Lasers erzeugt ist.

12. Tropfenabgabevorrichtung nach einem der vorhergehenden Ansprüche, wobei der zweite Abschnitt (5b) des Abflusskanals (5) durch herkömmliches Bohren, Formen oder Spritzgießen erzeugt ist.

13. Tropfenabgabevorrichtung nach einem der vorhergehenden Ansprüche, wobei der Innendurchmesser des proximalen Endes (5ap) des ersten Abschnitts (5a) des Abflusskanals (5) mindestens 5 %, vorzugsweise mindestens 10%, weiter bevorzugt mindestens 25 % größer als der Innendurchmesser des distalen Endes (5ad) des ersten Abschnitts (5a) des Abflusskanals (5) ist.

14. Verfahren oder Prozess zur Herstellung einer Tropfenabgabevorrichtung (1) nach einem der Ansprüche 1 bis 13, umfassend den Schritt Laserbohren des ersten Abschnitts (5a) des Abflusskanals.

15. Satz, umfassend
- eine Tropfenabgabevorrichtung (1) nach einem der Ansprüche 1 bis 13 und
- Anleitungen zur Verwendung der Tropfenabgabevorrichtung (1).

## Revendications

1. Distributeur de gouttes (1), comprenant :
- une partie récipient (IB) dont le volume intérieur est adapté pour être rempli en partie d'une phase liquide (2), une phase gazeuse (3) remplissant le reste du volume intérieur à pression ambiante, la partie récipient (1B) comportant une portion déplaçable (1C), et
- une partie compte-gouttes (1A) qui est en liaison physique et en communication fluidique avec le volume intérieur de la partie conteneur (1B), comprenant un canal de sortie (5) et qui relie le volume intérieur de la partie conteneur (1B) à l'environnement;
le conduit de sortie d'écoulement (5) comprenant
- une première portion (5a) pourvue d'une extrémité proximale (5ap) et d'une extrémité distale (5ad), chaque extrémité ayant le même diamètre intérieur, ou des diamètres intérieurs différents choisi indépendamment les uns des autres dans la gamme allant de 0,09 à 0,19 mm ; et
- une deuxième portion (5b) pourvue d'une extrémité proximale (5bp) et d'une extrémité distale (5bd) ; l'extrémité proximale (5bp) ayant un diamètre intérieur dans la gamme allant de 1 à 3 mm ; l'extrémité distale (5bd) ayant un diamètre intérieur dans la gamme de 0,1 à 3 mm, à condition que le diamètre intérieur à l'extrémité distale de la deuxième portion (5bd) soit supérieur au diamètre intérieur à l'extrémité proximale de la première portion (5ap) ;
et
- la première portion (5a) du conduit de sortie d'écoulement (5) étant réalisée par perçage laser.

2. Distributeur de gouttes selon la revendication 1, la somme de la longueur de la première portion (5a) et de la longueur de la deuxième portion (5b) étant égale à la longueur totale du conduit de sortie d'écoulement (5).

3. Distributeur de gouttes selon l'une quelconque des revendications précédentes, la première portion (5a) du conduit de sortie d'écoulement étant située à l'extrémité distale (4) du conduit de sortie d'écoulement.

4. Distributeur de gouttes selon l'une quelconque des revendications précédentes, l'extrémité distale (5bd) de la deuxième portion du conduit de sortie d'écoulement (5) étant adjacente à l'extrémité proximale (5ap) de la première portion (5a) du conduit de sortie d'écoulement.

5. Distributeur de gouttes selon l'une quelconque des revendications précédentes, le diamètre intérieur de l'extrémité proximale (5ap) de la première portion (5a) du conduit de sortie d'écoulement (5) étant supérieur au diamètre de l'extrémité distale (5ad) de la première portion (5a) du conduit de sortie d'écoulement (5).

6. Distributeur de gouttes selon l'une quelconque des revendications précédentes, le diamètre intérieur de la deuxième portion (5b) du conduit de sortie d'écoulement augmentant linéairement de l'extrémité distale (5bd) de la deuxième portion (5b) à l'extrémité proximale (5bp) de la deuxième portion.

7. Distributeur de gouttes selon l'une quelconque des revendications précédentes, la première portion (5a) et/ou la deuxième portion (5b) du conduit de sortie d'écoulement ayant une section transversale circulaire.

8. Distributeur de gouttes selon l'une quelconque des revendications précédentes, le diamètre intérieur à l'extrémité proximale (7) du conduit de sortie d'écoulement étant dans la gamme d'environ 2 à 3 mm.

9. Distributeur de gouttes selon l'une quelconque des revendications précédentes, le distributeur de gouttes (1) comprenant une composition liquide comme phase liquide (2), la phase liquide (2) comprenant un alcane semi-fluoré, de préférence choisi parmi F4H5 et F6H8.

10. Distributeur de gouttes selon l'une quelconque des revendications précédentes, la partie compte-gouttes (1A) étant en une matière polymère qui peut être percée par des techniques de perçage au laser et qui est utilisée pour le moulage par injection.

11. Distributeur de gouttes selon l'une quelconque des revendications précédentes, la première portion (5a) du conduit de sortie d'écoulement (5) étant réalisée par perçage au laser à l'aide d'un laser UV, d'un laser IR ou d'un laser au CO₂.

12. Distributeur de gouttes selon l'une quelconque des revendications précédentes, la deuxième portion (5b) du conduit de sortie d'écoulement (5) étant réalisée par perçage, moulage ou moulage par injection classique.

13. Distributeur de gouttes selon l'une quelconque des revendications précédentes, le diamètre intérieur de l'extrémité proximale (5ap) de la première portion (5a) du conduit de sortie d'écoulement (5) étant supérieur d'au moins 5 %, de préférence d'au moins 10 %, plus préférablement d'au moins 25 %, au diamètre intérieur de l'extrémité distale (5ad) de la première portion (5a) du conduit de sortie d'écoulement (5).

14. Procédé ou processus de fabrication d'un distributeur de gouttes (1) selon l'une quelconque des revendications 1 à 13, ledit procédé ou processus comprenant l'étape de perçage au laser de la première portion (5a) du conduit de sortie d'écoulement.

15. Kit comprenant
- un distributeur de gouttes (1) selon l'une quelconque des revendications 1 à 13 et
- des instructions d'utilisation du distributeur de gouttes (1).
